# EUROPEAN PATENT SPECIFICATION

(11) **EP 3 836 897 B1**
(45) Date of publication and mention of the grant of the patent: **01.02.2023**
(21) Application number: 19720875.4
(22) Date of filing: 30.04.2019
(51) Int. Cl.: A61K 9/00, A61K 9/46, A61K 9/68, A61K 9/10, A61K 31/09, A61P 11/10

(54) **COMPOSITIONS COMPRISING GUAIFENESIN AND ERIODICTYOL**
ZUSAMMENSETZUNGEN ENTHALTEND GUAIFENESIN UND ERIODICTYOL
COMPOSITIONS COMPRENANT GUAIFENESINE ET ÉRIODICTYOL

(30) Priority: 17.08.2018 US 201862719512 P
(43) Date of publication of application: 23.06.2021
(73) Proprietor: Symrise AG, 37603 Holzminden (DE)
(72) Inventor: HANS, Joachim, 37603 Holzminden (DE); LEY, Jakob, 37603 Holzminden (DE); GURNEY, Martin, Teterboro, New Jersey 07608 (US); SOMOZA, Veronika, 3400 Weidling (AT); BELTRAN, Leopoldo, 1190 Wien (AT)
(74) Representative: Global IP Europe Patentanwaltskanzlei
(86) International application number: PCT/EP2019/061024
(87) International publication number: WO 2020/035177

(56) References cited:
- EP-A1- 2 845 606
- LEY J P ET AL: "Evaluation of bitter masking flavanones from Herba Santa (Eriodictyon californicum (H.& A.) Torr., Hydrophyllaceae)", JOURNAL OF AGRICULTURAL AND FOOD CHEMISTRY, AMERICAN CHEMICAL SOCIETY, BOOKS AND JOURNALS DIVISION, US, vol. 53, no. 15, 27 July 2005 (2005-07-27) , pages 6061-6066, XP002696360, ISSN: 0021-8561, DOI: 10.1021/JF0505170 [retrieved on 2005-06-22] cited in the application

## Description

### Field of the invention

The present invention refers to a composition comprising guaifenesin and eriodictyol. In particular, the present invention refers to a composition comprising guaifenesin and eriodictyol, said composition for use as a medicament, in particular for use in the prevention and treatment of respiratory diseases, more particularly for treating a common cold, cough or catarrh. Moreover, the present invention relates to the use of guaifenesin and eriodictyol for the preparation of a pharmaceutical composition. Finally, the present invention relates to the use of eriodictyol for masking or inhibiting the bitter taste of guaifenesin compositions comprising guaifenesin.

### State of the art

Guaifenesin is a pharmaceutically effective compound which belongs to the class of mucokinetics. Mucokinetics are a class of drugs which aid in the clearance of mucus from the respiratory airways, lungs, bronchi and tracheae. Such drugs can be further categorized by their mechanism of action, namely as mucolytic agents, expectorants, surfactants, wetting agents (hypoviscosity agents) and abhesives. In general, the clearance ability of mucokinetics is hampered by bonding to surfaces (stickiness) and by the viscosity of mucous secretions in the lungs. In turn, the viscosity is dependent upon the concentration of mucoprotein in the secretions.

Expectorants and mucolytic agents are different types of medication, yet both are intended to promote drainage of mucus from the lungs. An expectorant increases bronchial secretion, and mucolytics help loosen viscous bronchial secretions. Expectorants reduce the thickness or viscosity of bronchial secretions thus increasing mucus flow that can be removed more easily through coughing.

Guaifenesin or compositions comprising guaifenesin or one or more of its isomers in pharmacologically effective amounts are administered orally and offered in liquid or solid form, as for example syrups, sprays, tablets, pills, capsules, etc. Regardless of the galenic presentation, the products suffer from the same disadvantage, that is a bitter taste. The bitter taste makes the oral administration unpleasant, and causes issues in patient compliance, especially when administered to children.

Typically, cough medicaments contain propylene glycol (1,2-propanediol) as a solvent. However, propylene glycol often enhances the bitterness of the active ingredients, so that products found in the market typically need to contain high amounts of sweeteners and/or aroma compounds to overcome or cover the bitter taste.

If pharmaceutical compositions have a bitter taste they may be mixed with a sweetening agent to mask the bitter taste. However, since some of the sweetening agents, have also a bitter taste of their own, the use of sweetening agents for masking or inhibiting bitter taste is limited.

Document EP 1 452 177 A1 refers to a bitterness masking agent for pharmaceutical formulations. It was found that sodium laurylsulfate can mask the bitter taste of some bitter tasting pharmaceutically active compounds, in particular epinastine or quinine comprising formulations.

Homoeriodictyol (HED) was described as a masking agent for guaifenesin in Ley et al., "Evaluation of bitter masking flavanones from Herba Santa (Eriodictyon californicum (H. & A.) Torr., Hydrphyllaceae)", J. Agric. Food Chem. 2005 (53), 6061 to 6066, Figure 5, Example 2. In a relatively high concentration of 200 ppm (200 mg/l HED), i.e. 0.2 mg/ml HED against 13 mg/ml guaifenesin, a reduction in guaifenesin bitterness of about 40 % was described.

Eriodictyol is described as a bitter masker in EP 1 258 200 A1 as exemplified with caffeine or in WO 2017/088936 A1 for omeprazole or pantoprazol. However, due to the very complex mechanisms behind human bitter taste reception and perception (Brockhoff, A.; Behrens, M.; Roudnitzky, N.; Appendino, G.; Avonto, C.; Meyerhof, W., Receptor agonism and antagonism of dietary bitter compounds, J. Neurosci., 2011, 31, 14775 to 14782), actually a potential bitter masking effect against other pharmaceutical active ingredients is not predictable due to missing or insufficient data on agonistic/antagonistic bitter receptor activities of guaifenesin.

EP 2 845 606 A1 discloses a medicament comprising at least one active pharmaceutical ingredient, 1,3-propandiol, and optionally a flavour modulating compound. The active pharmaceutical ingredient can be selected from acetaminophen, phenylephrine, guaifenesin, dextrometorphen, aspirin and/or ibuprofen and their mixtures. The flavour modulating compound can be preferably vanillin, pellitorine, homoeriodictyol, eriodictyol, hesperetin or phloretin.

The object of the present invention, therefore, has been to provide a composition comprising guaifenesin for the prevention and treatment of respiratory diseases with improved taste, in particular with reduced or eliminated bitter taste. Another object of the present invention has been to find other compounds which can mask or inhibit the bitter taste of guaifenesin of guaifenesin comprising compositions.

### Summary of the invention

Hence, in a first embodiment, the present invention relates to a composition, comprising
(a) guaifenesin or one or more of its salts or mixtures thereof, and
(b) eriodictyol or one or more of its salts or mixtures thereof; and
(c) optionally at least one pharmaceutically acceptable adjuvant or additive.

In another embodiment, the present invention relates to the use of the aforementioned composition for the preparation of pharmaceutical compositions and its use for the prevention and treatment of respiratory diseases.

In still another embodiment, the present invention relates to providing eriodictyol or its salts or mixtures thereof for masking or inhibiting the bitter taste of guaifenesin or one or more of its salts or mixtures thereof.

Preferred embodiments of the aforementioned composition are apparent from dependent claims.

### Detailed description of the invention

The present invention relates to a composition, comprising
(a) guaifenesin or one or more of its salts or mixtures thereof, and
(b) eriodictyol or one or more of its salts or mixtures thereof; and
(c) optionally at least one pharmaceutically acceptable adjuvant or additive.

Guaifenesin, also known as glyceryl guaiacolate, whose chemical name is 3-(2-methoxyphenoxy-)1,2-propanediol is an expectorant drug. The compound is represented by the following formula:

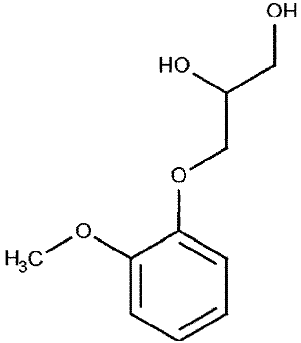

Guaifenesin exists in two stereo isomers, namely the R-enantiomer (see top of the below formulae) and the S-enantiomer (see bottom of the below formulae).

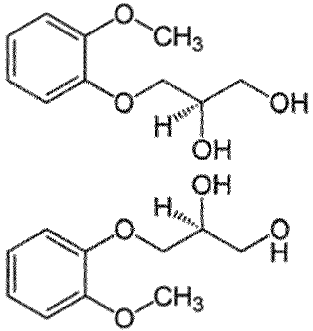

Throughout the present invention, the term "guaifenesin" includes either the R-enantiomer, the S-enantiomer, mixtures of the R- and S-enantiomers, or the racemate. In a preferred embodiment of the present invention, guaifenesin may be used as the racemate.

In particular, guaifenesin may be used as a mixture of enantiomers in the range of molar amounts of from 0.1 2S : 100 2R to 0.1 2R : 100 2S, as pure enantiomers, preferred as racemic mixture (50 : 50) or almost racemic mixtures of from 35 2S : 65 2R to 65 2R : 35 2S, preferably from 45 2S : 55 2R to 55 2R : 45 2S.

Furthermore, guaifenesin may be used in the form of its pharmaceutically acceptable salts, or mixtures thereof. In particular, preferred are the mono- or divalent salts and the ammonium salt. Among the salts, sodium, calcium, magnesium, potassium or ammonium are most preferred.

Guaifenesin is thought to act by thinning the mucus, loosening phlegm and bronchial secretion, and also by lubricating the irritated respiratory tract. By thinning the mucus, guaifenesin reduces the viscosity of the mucal secretion, and as a result increases the efficiency of the cough reflex and of ciliary action in removing accumulated secretions from the trachea and bronchi. The effect felt by an individual is that a nonproductive cough becomes more productive and less frequent. Unfortunately, guaifenesin has an unpleasantly bitter taste.

Surprisingly, it has been observed that patients, to whom the composition according to the present was administered, find that the composition has a much better taste, in particular a significantly reduced bitter taste, compared to a similar product comprising the same amount of guaifenesin, but comprising no eriodictyol. It was found that the bitter taste of guaifenesin may be masked or even inhibited by eriodictyol used according to the present invention.

In the context of the present invention, the term "masking" means a reduction, i.e. decrease, of the bitter taste. The term "inhibiting" means a complete suppression of the bitter taste.

In particular, when guaifenesin contained in a composition, preferably in a pharmacologically effective amount, is used in combination with eriodictyol, the bitterness is reduced by about 35 % or more, preferably by about 40 % or more. This effect is demonstrated in detail by the experimental data in Table 3.

An improvement in taste can be achieved by different strategies. Traditionally, an unpleasant bitter taste was diminished by the addition of pleasant flavouring substances, in order to merely cover the bitter taste. A second approach to suppress or inhibit bitter taste is to prevent contact of the bitter tasting compound with the bitter receptor in the mouth, specifically on the tongue of the person, to whom the composition containing the bitter tasting compound is administered. This may be achieved, for example, by encapsulation, molecular inclusion etc. of the bitter tasting compound. A third strategy of masking the bitter taste is the use of so-called taste receptor blockers (antagonists), which can reduce or inhibit the reaction of the taste receptor with the bitter tasting compound (agonist).

Up to now, it could not be determined yet according to which mechanism the masking action or inhibition of the bitter taste of guaifenesin by eriodictyol towards guaifenesin works.

Eriodictyol, whose chemical name is (2S)-/(2R)-2-(3,4-dihydroxyphenyl)-5,7-dihydroxy-4-chromanone is a flavonoid extracted e.g. from yerba santa (*Eriodictyon californicum*) a plant native to South West of North America/North of Mexico or can be obtained by deglycosylation from Eriocitrin/Neoeriocitrin occurring in (immature) fruits or leaves of bitter orange (*Citrus aurantium).* Alternatively, it can be produced by chemical or biotechnological oxidation of naringenin or by chemical or biotechnological demethylation from hesperitin or homoeriodictyol.

The compound is represented by the following formula:

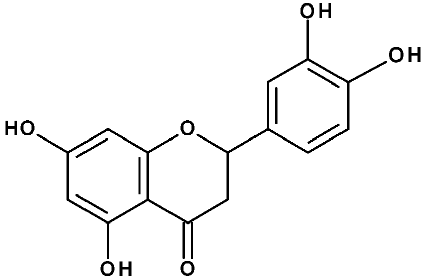

Eriodictyol exists in two stereo isomers, namely the 2R-enantiomer and the 2S-enantiomer. Throughout the present invention, the term "eriodictyol" includes either the 2R-enantiomer, the 2S-enantiomer, mixtures of the 2R- and 2S-enantiomers, or the racemate. In a preferred embodiment of the present invention, eriodictyol may be used as the racemate.

In particular, eriodictyol may be used as a mixture of enantiomers in the range of molar amounts of from 0.1 2S : 100 2R to 0.1 2R : 100 2S, as pure enantiomers, preferred as racemic mixture (50 : 50) or almost racemic mixtures of from 35 2S : 65 2R to 65 2R : 35 2S, preferably from 45 2S : 55 2R to 55 2R : 45 2S.

Preferably, eriodictyol is used in the form of its pharmaceutically acceptable salts, or mixtures thereof. In particular preferred are the mono- or divalent salts and the ammonium salt. Among the salts, sodium, calcium, magnesium, potassium or ammonium are most preferred.

The amount of eriodictyol used, including the amount of all enantiomers and/or salts or mixtures of eriodictyol used, depends on the amount of the bitter tasting ingredient guaifenesin contained in the composition according to the present invention. According to the invention, the amount sufficient to mask or even inhibit the bitter taste could be determined.

In preferred formulations, the composition of the present invention comprises guaifenesin in an amount of from 2000 to 20000 ppm. More preferred is an amount of from 5000 to 20000 ppm, and most preferred is an amount of from 5000 to 15000 ppm.

In preferred formulations, the composition of the present invention comprises eriodictyol in an amount of from 4 to 400 ppm. More preferred is an amount of from 10 to 200 ppm, and most preferred is an amount of from 10 to 50 ppm.

With regard to the bitter masking or inhibiting effect, the concentration of eriodictyol towards guaifenesin within the composition is decisive. Surprisingly, the bitter taste can be reduced or even inhibited most advantageously, if eriodictyol is used in an amount of 4 to 400 ppm, preferably in an amount of 10 to 50 ppm and guaifenesin is used in an amount of 2000 to 20000 ppm, preferably in an amount of 5000 to 15000 ppm.

The composition according to the present invention may comprise guaifenesin (compound (a)) and eriodictyol (compound (b)) in a ratio (w/w) from 5 : 1 to 5000 : 1. Preferably, the ratio (w/w) of guaifenesin (compound (a)) to eriodictyol (compound (b)) in the composition of the present invention is from 12.5 : 1 to 5000: 1. More preferably the ratio (w/w) of guaifenesin (compound (a)) to eriodictyol (compound (b)) in the composition of the present invention is from 12.5 : 1 to 3750 : 1. In a more preferred embodiment, in the composition of the present invention the ratio (w/w) of guaifenesin (compound (a)) to eriodictyol (compound (b)) is from 10 : 1 to 2000 : 1, more preferably from 25 : 1 to 2000 : 1 and most preferably from 25 : 1 to 1500 : 1. In still further preferred embodiments, the composition according to the present invention may comprise guaifenesin (compound (a)) and eriodictyol (compound (b)) in a ratio (w/w) from 40 : 1 to 2000 : 1, more preferably from 100 : 1 to 2000 : 1 and most preferably from 100 : 1 to 1500 : 1. The indication w/w stands for weight per weight of the composition.

Surprisingly, the bitter taste can still be further reduced or even inhibited, if the composition comprises homoeriodictyol (HED) whose chemical name is (2S)-/(2R)-5,7-Dihydroxy-2-(4-hydroxy-3-methoxyphenyl)-4-chromanone. Homoeriodictyol exists in two stereo isomers, namely the 2R-enantiomer and the 2S-enantiomer. Throughout the present invention, the term "homoeriodictyol" includes either the 2R-enantiomer, the 2S-enantiomer, mixtures of the 2R- and 2S-enantiomers, or the racemate.

Preferably, homoeriodictyol is used in the form of its pharmaceutically acceptable salts, or mixtures thereof. In particular, preferred are the mono- or divalent salts and the ammonium salt. Among the salts, sodium, calcium, magnesium, potassium or ammonium are most preferred.

If eriodictyol is used in combination with homoeriodictyol, the bitter masking effect can be increased significantly, as it is demonstrated by the following application examples. In a preferred embodiment of the present invention, there is even a synergistic effect, if eriodictyol and homoeriodictyol are used in combination in the inventive composition.

In addition to the above ingredients, the inventive formulation may also comprise further pharmaceutically effective ingredients including, but not limited to, analgetic agents or anti-inflammatory agents. The most preferred effective pharmaceutical ingredients are selected from the group consisting of acetaminophen, phenylephrine, dextromethorphan, aspirin, ibuprofen, diphenhydramine, antihistamines, naproxen sodium and their mixtures.

Moreover, the composition can contain one or more additional pharmaceutically effective agents including, but not limited to, an antitussive such as dextromethorphan hydrobromide, a decongestant such as phenylephrine hydrochloride, pseudoephedrine hydrochloride or ephedrine, an antihistamine such as chlorpheniramine maieate, brompheniramine maieate, phenindamine tartrate, pyrilamine maieate, doxylamine succinate, phenyitoloxamine citrate, diphenhydramine hydrochloride, promethazine, clemastine fumerate or fexofenadine or combinations thereof.

The composition according to the present invention usually contains at least two, often three, or even all of the afore-mentioned specified compounds. The amounts of the afore-mentioned pharmaceutically effective ingredients within the composition is about 0.1 to about 5 % by weight, preferably about 0.5 to about 3 % by weight and particularly about 1 to about 2 % by weight.

The composition can have an immediate release portion or a sustained release portion, such that the promotion of mucus secretion is therapeutically achieved for a period of approximately 12 hours.

In addition to guaifenesin or eriodictyol and the above additional pharmaceutically effective ingredients, the formulation according to the present invention optionally may comprise other pharmaceutically acceptable adjuvants and additives conventionally used for pharmaceutical preparations to be administered orally. These include but are not limited to inter alia carriers (e.g. microcrystalline cellulose), solvents (e.g. liquid polyethylene glycols), emulsifiers (e.g. sodium dodecylsulfate), dispersing agents (e.g. polyvinyl pyrrolidone), synthetic and natural biopolymers (e.g. albumin) stabilizers (e.g. antioxidants such ascorbic acid), colourings (e.g. inorganic pigments) and odour-correcting agents as well as taste-correcting agents that do not affect the masking or inhibition of the bitter taste.

Optionally, the formulation according to the present invention may comprise as adjuvant or additive sweetening agents to mask a quick-acting and a lasting bitter taste caused by guaifenesin. To mask the quick-acting bitter taste, cyclamate or its sodium salt, saccharin or its sodium salt, sucralose, acesulfam K, aspartame, thaumatin, neohesperedindihydrochalcone, advantam, neotam, glycyrrhicinic acid or its ammonium salt, or steviosides (e.g. stevioside, rebaudioside A, rebaudioside C, rebaudioside D, rebaudioside M, rubusoside) turned out to be effective.

Surprisingly, the bitter masking effect is even synergistic, if eriodictyol and the afore-mentioned sweeteners are used in combination in the inventive composition. In particular, a combination of eriodictyol and sweeteners such as cyclamate or its sodium salt, saccharin or its sodium salt, sucralose, acesulfam K, aspartame, neohesperedindihydrochalcone, glycyrrhicinic acid or its ammonium salt, stevioside, rebaudioside A, rebaudioside M or rubusoside turned out to be most effective in the masking or inhibiting the bitter taste of guaifenesin or a composition comprising guaifenesin.

Some of the aforementioned sweeteners, e.g. cyclamate or its sodium salt, saccharin or its sodium salt, acesulfam K, stevioside, rebaudioside A, exhibit a bitter (after)taste. Due to the known masking effect of homoeriodictyol against bitter taste of some sweeteners (Gaudette, N. J.; Delwiche, J. F.; Pickering, G. J., The contribution of bitter blockers and sensory interactions to flavour perception, Chemosensory Perception, 2015, 9 (1), 1 to 7), the combination of eriodictyol, homoeriodictyol, a sweetener and guaifenesin is in particular effective, as it is demonstrated by the following application examples, and preferred.

Another suitable group of sweet tasting compounds comprises sugars and sugar-derived polyols such as sucrose, glucose, fructose, allulose, trehalose, arabinose, D-sorbitol, palatinose, erythritol, xylitol, glycerine and D-mannitol.

The amount of these sweetening agents to mask the quick-acting bitterness depends of the agent used. In case of saccharin sodium, the amount is between 0.1 % by weight and 2.0 % by weight of a powder formulation. Preferably the amount is 0.8 % by weight. In case of aspartame the amount is between 1 % by weight and 30 % by weight of a powder formulation. Preferably the amount is 5 to 15 % by weight, most preferred it is 10 % by weight.

For masking the lasting bitterness, glycyrrhizinates were found to be highly effective. Among them, glycyrrhizinic acid and/or monoammonium glycyrrhizinate are the preferred ones. The most preferred one is monoammonium glycyrrhizinate.

The amount of monoammonium glycyrrhizinate in a powder formulation is from 0.1 % by weight to 3.0 % by weight. More preferred are 0.1 to 1% by weight, and most preferred is 0.6 % by weight.

Other kinds of adjuvants or additives are pH-adjusting agents to adjust the pH of the resulting composition to a value of preferably between 5 and 8, preferably 6 and 7. Among those agents are citric acid, succinic acid, tartaric acid, acetic acid, citrates, acetates, vitamin C, hydrochloric acid, carbonates, phosphates, disodium phosphate, monosodium phosphate, sodium, calcium, potassium and/or magnesium hydroxide. Preferred are buffer substances like disodium phosphate.

Further, other commonly used additives can optionally be added. Among these are binding agents such as for example hydroxypropylcellulose, methylcellulose, hydroxypropylmethylcellulose, hydroxyethylcellulose, starch, dextrin, gelatine and polyvinylpyrrolidone, preferably hydroxypropylcellulose, flow agents such as for example hydrated silicon dioxide or light anhydrous silicic acid or disintegrants such as for example starch, cellulose and derivatives, microcrystalline cellulose, alginates, bicarbonates or carbonates in combination with citric acid or tartaric acid.

According to another preferred embodiment of the present invention, the composition may contain at least one aroma compound, preferably at least one traditional flavour or a flavour modulating compound in order to complete and refine the taste and/or odour of the composition.

Such compounds can be chosen from synthetic flavouring liquid and/or oils derived from plants leaves, flowers, fruits and so forth, and combinations thereof. Representative flavouring liquids include: artificial, natural or synthetic fruit flavours such as eucalyptus, mint (peppermint, spearmint), lemon, orange, banana, grape, lime, apricot and grapefruit oils and fruit essences including apple, strawberry, cherry, orange, pineapple and so forth; bean and nut derived flavours such as coffee, cocoa, cola, peanut, almond and so forth; and root derived flavours such as liquorice or ginger.

The flavouring agent is preferably selected from the group consisting of essential oils and extracts, tinctures and balsams, such as, for example, anisole, basil oil, bergamot oil, bitter almond oil, camphor oil, citronella oil, lemon oil; Eucalyptus citriodora oil, eucalyptus oil, fennel oil, grapefruit oil, chamomile oil, spearmint oil, caraway oil, lime oil, mandarin oil, nutmeg oil (in particular nutmeg blossom oil), myrrh oil, clove oil, clove blossom oil, orange oil, oregano oil, parsley (seed) oil, peppermint oil, rosemary oil, sage oil (clary sage, Dalmatian or Spanish sage oil), star aniseed oil, thyme oil, vanilla extract, juniper oil (in particular juniper berry oil), wintergreen oil, cinnamon leaf oil; cinnamon bark oil, and fractions thereof, or constituents isolated therefrom.

It is of particular advantage if the flavoured composition according to the invention comprises at least one flavouring agent, preferably two, three, four, five, six, seven, eight or more flavouring agents chosen from the following group: menthol (preferably I-menthol and/or racemic menthol), anethole, anisole, anisaldehyde, anisyl alcohol, (racemic) neomenthol, eucalyptol (1,8-cineol), menthone (preferably L-menthone), isomenthone (preferably D-isomenthone), isopulegol, menthyl acetate (preferably L-menthyl acetate), menthyl propionate, carvone (preferably (-)-carvone, optionally as a constituent of a spearmint oil), methyl salicylate (optionally as a constituent of a wintergreen oil), eugenol acetate, isoeugenol methyl ether, beta-homocyclocitral, eugenol, isobutyraldehyde, 3-octanol, dimethyl sulfide, hexanol, hexanal, trans-2-hexenal, cis-3-hexenol, 4-terpineol, piperitone, linalool, 8-ocimenyl acetate, isoamyl alcohol, isovaleraldehyde, alpha-pinene, beta-pinene, limonene (preferably D-limonene, optionally as a constituent of an essential oil), piperitone, transsabinene hydrate, menthofuran, caryophyllene, germacrene D, cinnamaldehyde, mint lactone, thymol, gamma-octalactone, gamma-nonalactone, gamma-decalactone, (1,3E,5Z)undecatriene, 2-butanone, ethyl formate, 3-octyl acetate, isoamyl isovalerate, cis- and trans-carvyl acetate, p-cymol, damascenone, damascone, cis-rose oxide, trans-rose oxide, fenchol, acetaldehyde diethyl acetal, 1-ethoxyethyl acetate, cis-4-heptenal, cis-jasmone, methyl dihydrojasmonate, 2'-hydroxypropiophenone, menthyl methyl ether, myrtenyl acetate, 2-phenylethyl alcohol, 2-phenylethyl isobutyrate, 2-phenylethyl isovalerate, geraniol, nerol and viridiflorol.

Particular preferred flavouring compounds encompass menthol, cineol, eugenol, thymol, cinnamic aldehyde, peppermint oil, spearmint oil, eucalyptus oil, thyme oil, cinnamon oil, clove oil, spruce needle oil, fennel oil, sage oil, aniseed oil, star anise oil, chamomile oil, and caraway oil, and their mixtures. In the U.S., fruit flavours, especially cherry, grape, citrus and berry are preferred in the inventive compositions, especially for paediatric products.

The most preferred flavour modulating compounds are selected from the group consisting of vanillin, cis- or trans-pellitorine, hesperetin, matairesinol, and phloretin.

The aroma or flavouring compounds can be present in amounts of about 0.1 to about 5 % by weight, preferably about 0.5 to about 3 % by weight and in particular about 1 to about 2 % by weight.

For flavour solutions, 1,2-propandiol is often used as a solvent. 1,2-propandiol, however, exhibits some intrinsic bitterness. The bitterness of said solvent as well as the bitterness of guaifenesin in the inventive composition can be further reduced by replacing 1,2-propandiol in such a flavour solution by 1,3-propandiol, as it is demonstrated by the following application examples. Therefore, flavour formulations with 1,3-propandiol are preferred over the same formulations with 1,2-propandiol.

The composition according to the present invention may be administered orally and may be in the form of a liquid or in solid form.

In the case of liquid formulations, water, water-ethanol-mixture or ethanol as liquid/solvent is preferred. The liquid formulations are preferably selected from the group consisting of a syrup, a solution and a suspension. If the composition according to the present invention is administered in a solid form, the solid could be for example a powder, a capsule, a pill, a pastille or a hard-boiled candy form. The composition may also take the form of a gel or a gum.

The invention also may be used in aerosol formulations which are to be inhaled into the lungs. Among such formulations with water, water-ethanol-mixtures or ethanol as liquid medium or propellant driven are preferred.

As an alternative to powders, tablets may be used. In the case of a tablet, in particular, a chewable tablet or an effervescent tablet is preferred. The ingredients may be the same. In a preferred embodiment of the present invention, the powder formulation or composition is used as it is or can also be pressed to a tablet and - as needed - be dissolved in water, for example as an effervescent powder or tablet. In such an embodiment, the tablet - just as the powder formulation - may additionally comprise an effervescent agent such as bicarbonate.

In order to ensure that the patient can easily prepare a drinkable liquid formulation, the inventive composition or formulation can be delivered in separate packages. In these packages, water and the inventive composition or formulation are stored separately from each other. The package further allows both components to be mixed in an easy way.

As a consequence thereof, the present invention also relates to a kit of parts comprising two components, (a) the composition or formulation according to the invention and (b) water or alternatively a drinkable fluid like a juice, both components separated from each other.

To solve this issue, for example bottles having special caps can be used. Most often in such packages, the liquid solvent can be stored in a bottle of glass, plastic, metal and so on while the cap for closing the bottle comprises a chamber to take the composition or formulation of the present invention. Prior to use, the patient can take out the powder of the cap and mix it with the water in the bottle. This mixing process can either be done consciously, meaning the patient actively takes the powder and puts it into the water. In other embodiments, the patient can initiate the mixing process in a more automatic way by for example just screwing, pressing, shaking the cap or the bottle, in order to remove a barrier in the chamber containing the powder and by doing so allowing it to fall from the cap into the bottle.

Other, similar devices might be used, too. Besides, the inventive composition or formulation can be stored in an aluminium or plastic-bag or in an aluminium or plastic bottle. The thus stored powder then can be used with a pre-metered amount of water, stored in another package or with freshly filled drinking water, tap water or carbonized water.

The afore described composition is used as a medicament or for the preparation of a medicament. The medical composition is preferably used in the prevention or treatment of respiratory diseases, in particular the common cold, cough or catarrh which come along with bronchial secretions and mucolytics. The medical composition helps to loosen viscous bronchial secretions and to reduce the thickness or viscosity of bronchial secretions, thus increasing mucus flow that can be removed more easily through coughing.

Preferably the composition according to the present invention is used as an expectorant.

Additionally, the composition according to the present invention is used for the preparation of a pharmaceutical composition. These pharmaceutical compositions comprise both compositions which are non-prescription drugs and sold over the counter, and compositions which are only available on prescription.

Finally, the present invention relates to the use of eriodictyol or its salts or mixtures thereof for masking or inhibiting the bitter taste of guaifenesin or one or more of its salts or mixtures thereof or compositions comprising guaifenesin.

### Experimental

### Example 1: Effect of eriodictyol on guaifenesin-induced acid secretion in HGT-1 cells

Human gastric tumour cell line 1 (HGT-1; Laboisse C. L.; Augeron C.; Couturier-Turpin M. H.; Gespach C.; Cheret A. M.; Potet F., Characterization of a newly established human gastric cancer cell line HGT-1 bearing histamine H2-receptors, Canc. Res., 1982, 42, 1541 to 1548) is a suitable screening tool for detecting the interaction of bitter agents and bitter modulating substances (Liszt, K. I.; Hans, J.; Ley, J. P.; Kock, E.; Somoza, V., Characterization of bitter compounds via modulation of proton secretion in human gastric parietal cells in culture, J. Agric. Food Chem., 2018, 66 (10), 2295-2300.). Therefore, the effect of eriodictyol on guaifenesin-induced acid secretion in the HGT-1 cell line was determined to predict the modulation of guaifenesin-induced bitterness by eriodictyol. To this aim, HGT-1 cells were cultured under standard conditions and passaged at ca. 80 % confluence. Cells were then seeded into 96-well plates at a density of 10⁵/cm² and used for measurements when 80 % confluence was reached in the wells. HGT-1 cells were pre-loaded with (acetyloxy)methyl-3-(acetyloxy)-10-(dimethylamino)-3'-oxo-spiro[7H-benzo[c]xanthene-7,1'(3H)-isobenzofuran]-ar'-carboxylate and incubated in 130 mM NaCl 130, 4.7 mM KCI, 1.3 mM CaCl₂, 1.2 mM MgSO₄, 1.2 mM KH₂PO₄, 11.7 mM glucose,10 mM HEPES 10, 20 µM 4,4'-Diisothiocyanatostilbene-2,2'-disulfonic acid disodium and 20 µM 5-(N-Ethyl-N-isopropyl)amiloride at pH 7.4. Fluorescence recordings were performed with an excitation wavelength of 488 nm and monitoring the emission wavelengths of 580 nm and 640 nm, respectively, each with a 20 nm bandwidth. Recordings were performed over 5 min and raw data was converted into the log₂ transformed ratio of treated vs. untreated cells.

Co-incubation of 10 mM guaifenesin with 30 µM eriodictyol resulted in a significant decrease in proton secretory activity of HGT-1 cells from 10 min onwards compared to cells incubated with 10 mM guaifenesin alone (Table 1). Co-incubation of 10 mM guaifenesin with 30 µM homoeriodictyol did not exhibit such a pronounced inhibition of proton secretion, with only the time point at 10 min showing a significant decrease (p < 0.0071).

**Table 1**

| Timepoint [min] | Control | 10 mM guaifenesin | 10 mM guaifenesin + 3 µM eriodictyol | 10 mM guaifenesin + 30 µM eriodictyol |
|---|---|---|---|---|
| 0 | -0.0254±0.0230 | -0.3130±0.0306 | -0.3389±0.0274 | -0.2324±0.0297 |
| 5 | -0.0117±0.0204 | -0.2720±0.0225 | -0.3351±0.0246 | -0.2396±0.0252 |
| 10 | - 0.0233±0.0179 | -0.3283±0.0253 | -0.2874±0.0241 | -0.1630±0.0308*** |
| 15 | - 0.0190±0.0215 | -0.2945±0.0277 | -0.2595±0.0264 | -0.1472±0.0302** |
| 20 | -0.0008±0.0180 | -0.2885±0.0266 | -0.2622±0.0286 | -0.1496±0.0269** |
| 25 | -0.0119±0.0217 | -0.3135±0.0260 | -0.3259±0.0279 | -0.1478±0.0283*** |
| 30 | -0.0079±0.0215 | -0.3241±0.0280 | -0.3237±0.0296 | -0.1886±0.0272* |

The data in Table 1 are presented as mean ± SEM derived from 7 biological replicates and 14 to 21 technical replicates. Outliers were identified using the Nalimov test, statistical significance was tested one-way ANOVA with Tukey's range test for multiple comparisons and Bonferroni correction, establishing the threshold for significance at p < 0.0071. Accordingly, data are marked with * (p < 0.0071), ** (p < 0.0014) or *** (p < 0.00014). Control: untreated cells

**Table 2**

| Timepoint [min] | Control | 10 mM guaifenesin | 10 mM guaifenesin + 3 µM homoeriodictyol | 10 mM guaifenesin + 30 µM homoeriodictyol |
|---|---|---|---|---|
| 0 | -0.0261±0.0178 | -0.2712±0.0190 | -0.2514±0.0260 | -0.2845±0.0235 |
| 5 | -0.0165±0.0230 | -0.2170±0.0217 | -0.1923±0.0304 | -0.1702±0.0265 |
| 10 | 0.0008±0.0222 | -0.2543±0.0268 | -0.2547±0.0320 | -0.1272±0.0263* |
| 15 | -0.0021±0.0227 | -0.2518±0.0274 | -0.2538±0.0317 | -0.1934±0.0259 |
| 20 | -0.0223±0.0225 | -0.2081±0.0243 | -0.2677±0.0264 | -0.2282±0.0260 |
| 25 | -0.0146±0.0222 | -0.2454±0.0265 | -0.2472±0.0296 | -0.1601±0.0206 |
| 30 | -0.0134±0.0213 | -0.2730±0.0241 | -0.2493±0.0300 | -0.2187±0.0244 |

The data in Table 2 are presented as mean ± SEM derived from 7 biological replicates and 14 to 21 technical replicates. Outliers were identified using the Nalimov test, statistical significance was tested one-way ANOVA with Tukey's range test for multiple comparisons and Bonferroni correction, establishing the threshold for significance at p < 0.0071. Accordingly, data are marked with * (p < 0.0071), ** (p < 0.0014) or *** (p < 0.00014). Control: untreated cells

### Example 2: Sensory effect of eriodictyol on guaifenesin-induced bitterness perception

To verify the effect of eriodictyol on guaifenesin-induced bitterness perception, an aqueous solution of 13 g/l guaifenesin (1) was tested against an aqueous solution containing 13 g/l guaifenesin and 20 mg/l eriodictyol (2) or 100 mg/l homoeriodictyol (3). The test was conducted as a randomized comparison test in a trained sensory panel (n = 20 to 30) via a sip and spit procedure. The panelists tasted both solutions in a blinded fashion and rated bitterness on a scale of 0 to 100. The average values of the bitter ratings are shown in table 3.

**Table 3**

| Example | | Bitter rating | Reduction versus control |
|---|---|---|---|
| 1 | 13 g/l guaifenesin | 76 | |
| 2 | 13 g/l guaifenesin + 20 mg/l eriodictyol (racemic) | 46 | -39 |
| 3 | 13 g/l guaifenesin + 100 mg/l homoeriodictyol (racemic) | 59 | -17 |

### Application examples:

Application example 1: Syrup containing guaifenesin and eriodictyol

Preparation of a syrup containing 100 mg guaifenesin/dose according to the following recipe:

| Constituent | Content in % (w/w) | | |
|---|---|---|---|
| | A | B | C |
| Guaifenesin | 1.25 | 1.25 | 1.25 |
| 1,2-propandiol | 23 | 23 | 23 |
| Glycerol | 8 | 8 | 8 |
| Sorbitol | 13.15 | 13.15 | 13.15 |
| Xanthan Gum | 0.2 | 0.2 | 0.2 |
| Symrise Aroma Type Strawberry | 0.4 | - | - |
| Symrise Aroma Type Strawberry containing 0.36 % eriodictyol with 1,2-propandiol as solvent | - | 0.4 | - |
| Symrise Aroma Type Strawberry containing 0.36 % eriodictyol with 1,3-propandiol as solvent | - | - | 0.4 |
| Water | add to 100 | add to 100 | add to 100 |

All syrups were thoroughly mixed until all constituents were dissolved completely. Syrup B and Syrup C was significantly less bitter than syrup A. Syrup C was even less bitter than Syrup B.

Application example 2: Powder formulation with guaifenesin and eriodictyol or eriodictyol/homoeriodictyol

Preparation of a powder containing 75 mg guaifenesin/dose according to the following recipe:

| Constituent | Content in % (w/w) | | |
|---|---|---|---|
| | A | B | C |
| Guaifenesin | 1.5 | 1.5 | 1.5 |
| Citric acid | 3 | 3 | 3 |
| Saccharin sodium | 2.5 | 2.5 | 2.5 |
| Ascorbic acid | 1 | 1 | 1 |
| Symrise Aroma Type Lemon | 0.8 | - | - |
| Symrise Aroma Type Lemon containing 0.18 % eriodictyol | - | 0.8 | - |
| Symrise Aroma Type Lemon containing 0.12 % eriodictyol and 0.20 % homoeriodictyol | - | - | 0.8 |
| Sucrose | add to 100 | add to 100 | add to 100 |

All powders were dry mixed and sieved. Powder B was significantly less bitter than powder A. Powder C was even less bitter than powder B.

Application example 3: Chewable tablets (2 g) with 100 mg guaifenesin/dose

| Constituent | Content in % (w/w) | |
|---|---|---|
| | A | B |
| Guaifenesin | 1 | 1 |
| Calcium carbonate | 25 | 25 |
| Magnesium stearate | 0.5 | 0.5 |
| Citric acid | 0.75 | 0.75 |
| Symrise Aroma Type Orange | 0.8 | - |
| Symrise Aroma Type Orange containing 0.18 % eriodictyol | - | 0.8 |
| Sucralose | 0.075 | 0.075 |
| Dextrose | add to 100 | add to 100 |

All constituents were thoroughly mixed and left to rest for 1 to 2 hrs, then tablets were pressed. Formulation B was less bitter than formulation A.

Application example 4: Effervescent tablets with 50 mg guaifenesin per dose

| Constituent | Content in % (w/w) | | |
|---|---|---|---|
| | A | B | C |
| Guaifenesin | 1 | 1 | 1 |
| Sorbitol | 8.4 | 8.4 | 8.4 |
| Sodium cyclamate | 1.5 | 1.5 | 1.5 |
| Sucralose | 0.25 | 0.25 | 0.25 |
| Symrise Aroma Type Lemon | 0.4 | - | - |
| Symrise Aroma Type Lemon containing 0.36 % eriodictyol | - | 0.4 | - |
| Symrise Aroma Type Lemon | - | - | 0.4 |
| containing 0.15 % eriodictyol and 0.20 % homoeriodictyol | | | |
| 1,3-propandiol | 0.625 | 0.625 | 0.625 |

All constituents are thoroughly mixed and then filled to 100 % with a premix of sodium hydrogencarbonate and citric acid (in a ratio of 1 : 1.36 (w/w)). The mixture is left to rest for 1 to 2 hrs and then sieved and subsequently pressed into tablets. Formulation B exhibited reduced bitterness compared to formulation A. Formulation C was even less bitter than formulation B.

Application example 5: Fruit gums containing 75 mg guaifenesin/dose

| Constituent | Content in % (w/w) | | |
|---|---|---|---|
| | A | B | C |
| Guaifenesin | 1.5 | 1.5 | 1.5 |
| Gelatine 240 Bloom | 7.6 | 7.6 | 7.6 |
| Saccharose | 34.50 | 34.50 | 34.50 |
| Glucose syrup, DE 40 | 31.89 | 31.89 | 31.89 |
| Iso Syrup C* Tru Sweet 01750 (Cerestar GmbH) | 1.50 | 1.50 | 1.50 |
| Yellow and red coloring | 0.01 | | |
| Citric acid | 0.2 | | |
| Symrise Aroma Type Raspberry | 0.4 | - | - |
| Symrise Aroma Type Raspberry | - | 0.4 | - |
| containing 0.36 % eriodictyol with 1,2-propandiol as solvent | | | |
| Symrise Aroma Type Raspberry containing 0.36 % eriodictyol with 1,3-propandiol as solvent | - | - | 0.4 |
| Water | add to 100 | add to 100 | add to 100 |

Formulation B and C exhibited considerably reduced bitterness compared to formulation A. Formulation C was even less bitter than formulation B.

### Application example 6: Throat candies with a liquid-viscous core filling (centre-filled hard candy)

| Constituent | Content in % (w/w) | |
|---|---|---|
| | **A** | **B** |
| **Part A (shell) (80 % of the candy)** | | |
| Sugar (sucrose) | add to 100 | add to 100 |
| Glucose syrup (solids content 80 %) | 41.51 | 49.37 |
| Mixture X1 of EP 2 187 871 B1 | 0.75 | 0.95 |
| I-Menthol | 0.10 | - |
| Lemon oil | 0.10 | 0.10 |
| Citric acid | - | 0.91 |
| Total A: | 100 | 100 |

| **Part B (core) (20% of the candy)** | | |
|---|---|---|
| High fructose corn syrup (content of solid sugars 85 %, close to 15 % water) | add to 100 | add to 100 |
| Glycerol | 15.0 | 15.0 |
| Lecithin | 0.02 | 0.02 |
| Guaifenesin | 5 | 5 |
| Pellitorin | 0.10 | 0.25 |
| Capsaicin | 0.05 | - |
| Homovanillic acid ethylester analogous to EP 2 932 858 | - | 0.50 |
| Red dyestuff, as a 5 % strength aqueous solution | 0.20 | 0.20 |
| Eriodictyol, 5 % in 1,3-propandiol | 0.1 | 0.1 |
| Total B: | 100 | 100 |

Bonbons having a liquid-viscous core were prepared in accordance with the processes described in US 6,432,441 (Example 1) and in US 5,458,894 and US 5,002,791. The two parts A and B were processed separately to bases for the shell (Part A) and the core (Part B). The filled throat candies obtained by means of co-extrusion acted against coughing, sore throat and hoarseness when consumed by affected persons.

## Claims

1. Composition, comprising
(a) guaifenesin or one or more of its salts or mixtures thereof, and
(b) eriodictyol or one or more of its salts or mixtures thereof; and
(c) optionally at least one pharmaceutically acceptable adjuvant or additive.

2. The composition according to claim 1, comprising guaifenesin in an amount of from 2000 to 20000 ppm, preferably in an amount of from 5000 to 20000 ppm, more preferably in an amount of from 5000 to 15000 ppm.

3. The composition according to any one of the preceding claims, comprising eriodictyol in an amount of 4 to 400 ppm, preferably in an amount of from 10 to 200 ppm, more preferably in an amount of from 10 to 50 ppm.

4. The composition according to any one of the preceding claims, comprising component (a) and component (b) in a ratio (w/w) of from 5 : 1 to 5000 : 1, in particular from 12.5 : 1 to 5000 : 1 or from 12.5 : 1 to 3750 : 1.

5. The composition according to any one of the preceding claims, further comprising homoeriodictyol or its salts or mixtures thereof.

6. The composition according to any one of the preceding claims, further comprising one or more pharmaceutically effective compounds, in particular analgesic agents, anti-inflammatory agents, antitussives, decongestants, antihistamines, or combinations thereof.

7. The composition according to any one of the preceding claims, wherein the at least one adjuvant is selected from the group consisting of carriers, solvents, emulsifiers, dispersing agents, synthetic and natural biopolymers, stabilizers, colourings, pH-adjusting agents, flow agents, disintegrants, odour-correcting agents and taste-correcting agents.

8. The composition according to any one of the preceding claims for oral application.

9. The composition according to any one of the preceding claims in the form of a liquid, in particular a syrup, in solid form, in particular a powder or a tablet, in particular a chewable tablet, an effervescent tablet, a capsule, a gel, a gum or an aerosol.

10. The composition according to any one of the preceding claims for use as a medicament.

11. The composition according to any one of the claims 1-9 for use in the prevention or treatment of respiratory diseases, in particular cold, cough or catarrh.

12. The composition according to any one of the claims 1-9 for use as an expectorant.

13. Use of eriodictyol or its salts or mixtures thereof for masking or inhibiting the bitter taste of guaifenesin or one or more of its salts or mixtures thereof or compositions comprising guaifenesin.

## Patentansprüche

1. Zusammensetzung, die aufweist:
(a) Guaifenesin oder eines oder mehrere seiner Salze oder Mischungen davon,
und
(b) Eriodictyol oder eines oder mehrere seiner Salze oder Mischungen davon;
und
(c) optional mindestens ein pharmazeutisch annehmbares Hilfsmittel oder Additiv.

2. Zusammensetzung nach Anspruch 1, die Guaifenesin in einer Menge von 2000 bis 20000 ppm, vorzugsweise in einer Menge von 5000 bis 20000 ppm, besonders bevorzugt in einer Menge von 5000 bis 15000 ppm aufweist.

3. Zusammensetzung nach einem der vorhergehenden Ansprüche, die Eriodictyol in einer Menge von 4 bis 400 ppm, vorzugsweise in einer Menge von 10 bis 200 ppm, besonders bevorzugt in einer Menge von 10 bis 50 ppm aufweist.

4. Zusammensetzung nach einem der vorhergehenden Ansprüche, die die Komponente (a) und die Komponente (b) in einem Verhältnis (w/w) von 5 : 1 bis 5000 : 1, insbesondere von 12,5 : 1 bis 5000 : 1 oder von 12,5 : 1 bis 3750 : 1 aufweist.

5. Zusammensetzung nach einem der vorhergehenden Ansprüche, die ferner Homoeriodictyol oder seine Salze oder Mischungen davon aufweist.

6. Zusammensetzung nach einem der vorhergehenden Ansprüche, die ferner eine oder mehrere pharmazeutisch wirksame Verbindungen aufweist, insbesondere Analgetika, Entzündungshemmer, Antitussiva, Dekongestiva, Antihistaminika oder Kombinationen davon.

7. Zusammensetzung nach einem der vorangehenden Ansprüche, wobei der mindestens eine Hilfsstoff aus der Gruppe ausgewählt ist, die aus Trägern, Lösungsmitteln, Emulgatoren, Dispergiermitteln, synthetischen und natürlichen Biopolymeren, Stabilisatoren, Farbstoffen, pH-Einstellmitteln, Fließmitteln, Sprengmitteln, Geruchskorrekturmitteln und Geschmackskorrekturmitteln.

8. Zusammensetzung nach einem der vorangehenden Ansprüche zur oralen Anwendung.

9. Zusammensetzung nach einem der vorhergehenden Ansprüche in Form einer Flüssigkeit, insbesondere eines Sirups, in fester Form, insbesondere eines Pulvers oder einer Tablette, insbesondere einer Kautablette, einer Brausetablette, einer Kapsel, eines Gels, eines Gummis oder eines Aerosols.

10. Zusammensetzung nach einem der vorangehenden Ansprüche zur Verwendung als Arzneimittel.

11. Zusammensetzung nach einem der Ansprüche 1 bis 9 zur Verwendung bei der Vorbeugung oder Behandlung von Atemwegserkrankungen, insbesondere Erkältung, Husten oder Katarrh.

12. Zusammensetzung nach einem der Ansprüche 1 bis 9 zur Verwendung als Expektorans.

13. Verwendung von Eriodictyol oder seinen Salzen oder Mischungen davon zur Maskierung oder Hemmung des bitteren Geschmacks von Guaifenesin oder einem oder mehreren seiner Salze oder Mischungen davon oder Zusammensetzungen, die Guaifenesin aufweisen.

## Revendications

1. Composition, comprenant
(a) de la guaïfénésine, ou un ou plusieurs de ses sels, ou des mélanges de ceux-ci,
et
(b) de l'ériodictyol, ou un ou plusieurs de ses sels, ou des mélanges de ceux-ci ;
et
(c) facultativement, au moins un adjuvant ou additif pharmaceutiquement acceptable.

2. Composition selon la revendication 1, comprenant de la guaïfénésine dans une teneur comprise entre 2000 et 20000 ppm, préférentiellement dans une teneur comprise entre 5000 et 20000 ppm, tout particulièrement dans une teneur comprise entre 5000 et 15000 ppm.

3. Composition selon l'une des revendications précédentes, comprenant l'ériodictyol dans une teneur comprise entre 4 et 400 ppm, préférentiellement dans une teneur comprise entre 10 et 200 ppm, tout particulièrement dans une teneur comprise entre 10 et 50 ppm.

4. Composition selon l'une des revendications précédentes, comprenant le composant (a) et le composant (b) suivant un rapport (w/w) compris entre 5 : 1 et 5000 : 1, en particulier entre 12,5 : 1 et 5000 : 1 ou entre 12,5 : 1 et 3750 : 1.

5. Composition selon l'une des revendications précédentes, comprenant en outre de l'homoériodictyol, ou ses sels, ou des mélanges de ceux-ci.

6. Composition selon l'une des revendications précédentes, comprenant en outre un ou plusieurs composés pharmaceutiquement efficaces, en particulier des agents analgésiques, des agents anti-inflammatoires, antitussifs, décongestifs, antihistaminiques, ou des combinaisons de ceux-ci.

7. Composition selon l'une des revendications précédentes, où ledit au moins un adjuvant est sélectionné dans le groupe comprenant des excipients, des solvants, des émulsifiants, des agents dispersants, des biopolymères synthétiques et naturels, des stabilisants, des colorants, des agents correcteurs d'acidité, des agents fluidifiants, des délitants, des agents anti-malodorants et des agents correcteurs de goût.

8. Composition selon l'une des revendications précédentes, destinée à une application orale.

9. Composition selon l'une des revendications précédentes, sous forme liquide, en particulier de sirop, sous forme solide, en particulier de poudre ou de comprimé, en particulier de comprimé à croquer, de comprimé effervescent, de capsule, de gélule, de gomme ou d'aérosol.

10. Composition selon l'une des revendications précédentes, destinée à être utilisée comme médicament.

11. Composition selon l'une des revendications 1 à 9, destinée à être utilisée pour la prévention ou le traitement d'affections respiratoires, en particulier rhume, toux ou catarrhe.

12. Composition selon l'une des revendications 1 à 9, destinée à être utilisée comme expectorant.

13. Utilisation de l'ériodictyol, ou de ses sels, ou de mélanges de ceux-ci, pour masquer ou inhiber l'amertume de la guaïfénésine, ou d'un ou de plusieurs de ses sels, ou de mélanges de ceux-ci, ou de compositions comprenant de la guaïfénésine.
